# EUROPEAN PATENT SPECIFICATION

(11) **EP 0 320 541 B1**
(45) Date of publication and mention of the grant of the patent: **31.08.1994**
(21) Application number: 87310969.8
(22) Date of filing: 14.12.1987
(51) Int. Cl.: A61B 19/04

(54) **Penetration-resistant surgical glove**
Eindringresistent-Operationshandschuh
Gant chirurgical résistant à la pénétration

(43) Date of publication of application: 21.06.1989
(73) Proprietor: Seid, Arnold Steven, Santa Monica California 90402 (US)
(72) Inventor: Seid, Arnold Steven, Santa Monica California 90402 (US)
(74) Representative: Senior, Alan Murray

(56) References cited:
- EP-A- 0 118 898
- WO-A-81/00345
- DE-U- 8 610 809
- GB-A- 2 033 731
- GB-A- 2 100 580
- US-A- 3 633 216
- US-A- 4 742 578
- Neue polymere Werkstoffe für die industrielle Anwendung, 243-256 Elias-Wohwinkel, Verlag Hanser 1983

## Description

The present invention relates to surgical appliances and, in particular, to surgical gloves designed to resist penetration by sharp needles or instruments.

The conventional surgical glove is composed of extremely thin latex or synthetic rubber designed to fit tightly and closely over the surgeon's hand, thereby providing a maximum of tactile sensitivity and ease of manipulation of the thumb and fingers. The glove serves as a protective shield to prevent the passage of contamination either from the hands of the surgeon to the patient or from the wound of the patient to the surgeon. While the thickness of surgical gloves may vary from one type or manufacture to another, the orthopedic surgeon's glove being composed of thicker latex, all are susceptible to puncture or rupture during the course of an operation.

In those cases where the surgeon is particularly concerned with glove puncture, he may employ the practice of double gloving; i.e., the wearing of two pairs of gloves. The protection afforded by this practice is, however, of minimum value providing, at most, only twice the protection of a single glove.

While attempts at solving the problems of rupture and penetration have included double dipping in liquid latex to increase the thickness of the glove, such attempts specifically proposing an increase in the thickness of the thumb and forefinger portions of the glove, the problem persists.

The present invention is directed to an improved surgical glove that is a combination of latex rubber and a thin, pliable and limp overlay of material composed of tightly interlaced fibers of high strength adhesively attached or bonded to the palmar or front face of the glove. The improved glove provides an appreciable improvement in the resistance to rupture and penetration, such as caused by sharp instruments, while retaining, to a large extent, the feel and tactile sensitivity of the conventional surgical glove.

Accordingly, the principal object of the present invention is to provide an improved penetration-resistant surgical glove which comprises a thin and pliable surgical glove reinforced in the palmar or front surface area with a tightly interwoven, penetration-resistant material to minimise the risks of puncture or rupture.

A further object is to provide a thin, pliable and lightweight reinforced surgical glove having a touch and feel similar to that of conventional latex surgical gloves.

An additional object is to provide a surgical glove providing increased protection to both physician and patient from exposure to contamination.

US-A-3 633 216 discloses a rubber surgical glove in accordance with the prior art portion of claim 1. This glove has, as an overlay of a portion thereof, an additional layer of latex material to provide localised additional thickness to reduce the possibility of puncture. The present invention as defined in claim 1, uses a specific overlay which is non-elastic, that is it is not stretchable, with this overlay being specifically constructed of interwoven high strength yarns so as to provide substantial improvement in the resistance to penetration whilst still being such as to permit the surgeon using the gloves to retain his feel. Because of the lack of elasticity, the woven overlay material is applied solely to the front of the glove, where it is most subject to the chance of penetration by sharp instruments, with the rear of the glove being left elastic to permit its close fitting to the hand of the surgeon.

Whilst it has been proposed in DE-U-8 610 809 to provide an underglove with a protective fabric providing an increased resistance to cutting, there is no appreciation in this prior glove as to how the technique could be adapted for use in a surgical glove where the problems are very acute. This prior glove has a fabric glove with an elastic knitted outer cut-resistant fabric which would not be suitable for use with a surgical glove both due to its lack of resistance to penetration by a sharp pointed instrument, such as a needle, and due to the complete inability of it being able to provide the necessary feel to a surgeon using the glove.

In its preferred form, with a semi-transparent coating to the overlay, the surgical glove is designed to reveal, upon visual inspection, that a rupture or penetration has occurred.

The present invention will be more fully understood from the following detailed exemplary description in connection with the accompanying drawings, wherein:
Fig. 1 is a front or palmar view of the preferred embodiment of the penetration-resistant glove of the invention as it appears upon a conventional porcelain form of the human hand.
Fig. 2 is an enlarged side view of the thumb portion of the glove of Fig. 1, illustrating the partial wrap-around of the penetration-resistant material.
Fig. 3 is an enlarged cross-sectional view of the index or forefinger portion of the glove of Fig. 1 taken along the lines 3-3 as viewed from the right side of Fig. 1.
Fig. 4 is an enlarged cross-sectional view of the index finger portion of the glove of Fig. 1 taken along the line 4-4 of Figure 1.

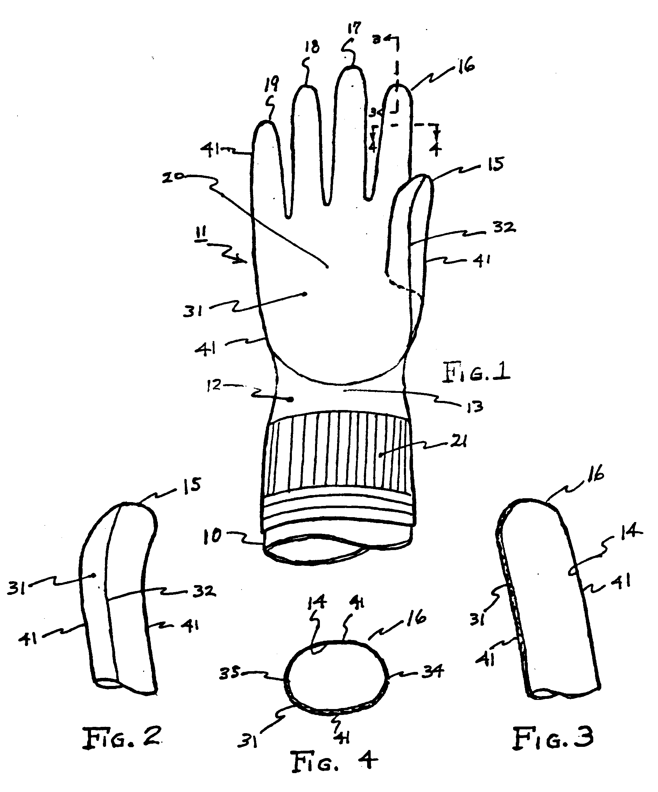

Referring to Fig. 1, the penetration-resistant glove 11 of the invention is shown situated upon a conventional hollow porcelain form 10 in the shape of the human hand. Hollow porcelain form 10 provides a base or foundation upon which glove 11 may be manufactured.

Fitted upon porcelain form 10 and in physical contact therewith is a first or foundation surgical glove 12 composed of thin, resilient and elastic material, such as latex or synthetic rubber. Foundation glove 12 has a front face or palmar surface 13, a rear surface 14, and includes thumb portion 15, finger portions 16-19, and palm portion 20, as shown. Glove 12 also includes cuff portion 21 for surrounding the wrist.

Foundation glove 12 may be pre-manufactured and manually placed or fitted upon porcelain form 10, or it may be manufactured directly upon form 10 by the conventional process of dipping the form in liquid latex, withdrawing, drying and vulcanizing. Glove 12 may have a thickness of 0.076 to 0.13 mm (three- to five-thousandths of an inch).

Attached to the front face or palmar surface 13 of foundation glove 12 is a thin overlay of penetration-resistant cloth material 31, pre-cut in the shape of the thumb 15, fingers 16-19 and palm portion of glove 12. Overlay material 31, shown as shaded or crosshatched lines, is composed of a very large number of tightly interlaced fibers or filaments of high tensile strength, as will be more fully described hereinbelow.

Overlay material 31 is pre-cut from the thin, penetration-resistant cloth material and is sized somewhat wider than thumb 15, fingers 16-19 and palm portion 20 of glove 12. This wider sizing enables overlay material 31 to be wrapped partially about the left and right sides of thumb 15 and fingers 16-19, as illustrated in Figs. 1, 2 and 4.

To attach overlay material 31 to the front face 13 of glove 12, a thin, surface coat of adhesive or contact cement is applied evenly to the rear surface of the material, as by spraying or spreading. This thin coat of adhesive may be applied to the rear surface after overlay material 31 has been pre-cut, or it say be applied to the cloth material while still in its flat cloth form, the applied adhesive coat being protected by a non-adherent film or backing.

The pre-cut, shaped, adhesively coated overlay material 31 is carefully aligned with the matching front surface of thumb 15, fingers 16-19, and palm portion 20 of glove 12 and attached thereto. Proper alignment assures that the edges of overlay material 31 will extend partially about thumb 15 and fingers 16-19 to the middle of each side of thumb 15 and fingers 16-19, as may be seen in Figs. 1, 2 and 4.

The enlarged side view of Fig. 2 shows the edge 32 of overlay material 31 positioned and affixed along the middle of the right side of the thumb portion 15. It will be appreciated that the peripheral edges of pre-cut overlay material 31 for the finger portion 16-19, as well as for the left side of thumb portion 15, will be positioned and affixed along the middle of the sides of the finger and thumb portions, respectively.

Figs. 3 and 4 are enlarged sectional views of the index or forefinger portion 16 of glove 11. In these two views, the thickness of overlay material 31 is shown exaggerated for the purpose of illustration. The rear surface 14 of index finger portion 16 of glove 12 is identified, in Figs. 3 and 4, as the inside surface, for reasons that will become apparent hereinafter. Overlay material 31 extends substantially to the tip of index finger portion 16, as seen in Fig. 3, and peripheral edges 34 and 35 of overlay 31 extend around the left and right sides to the positions shown in Fig. 4.

An acceptable surgical glove must be thin, pliable and tight-fitting to provide the required tactile sensitivity and ease of manipulation. It is necessary, therefore, for overlay material 31 of the improved surgical glove 11 to have a thinness and pliability approaching that of latex rubber. To achieve an improved penetration-resistant surgical glove, it is necessary to produce a special material having the required thinness and pliability as well as an additional toughness needed to prevent penetration by sharp-pointed instruments.

One representative example of a suitable material for overlay 31 is a special cloth obtainable from Burlington Industries, 1345 Avenue of the Americas, New York, New York 10019, and having the specification listed below:
- Material:: High-density interwoven nylon yarn
- Style No.:: 55116
- Weight:: 51 g per m² (1.5 oz per yd²)
- Thickness:: Approximately 0.076 mm (0.003 in)
- Type of Weave:: Plain
- Warp:: 30 denier nylon yarn, duPont type 285, zero twist
94.5 yarns per cm (240 yarns per inch)
26 filaments per yarn
- Fill:: 20 denier nylon yarn, duPont type 285, zero twist
78.7 yarns per cm (200 yarns per inch)
7 filaments per yarn
- Color:: White
The high-density interwoven cloth identified above possesses a resistance to penetration by a sharp-pointed needle three to five times greater than that of latex rubber of the same thickness. Surgical glove 11, with its adhesively attached overlay material 31 composed of this special interwoven high-density nylon, provides a resistance to penetration approximately five to seven times greater than the conventional surgeon's latex rubber glove.

The above-identified high-density nylon material forming overlay 31 is substantially non-elastic. Thus, while it possesses the desired thinness, i.e., 0.076 mm (0.003 in) extremely pliable and limp, it does not stretch. For this reason overlay material 31 is adhesively attached over only the front face or palmar surface 13 and partially around the left and right sides of thumb portion 15 and finger portions 16-19. The rear surface 14 of glove 11 and the sides of thumb portion 15 and finger portions 16-19 not covered by overlay 31 remain elastic, thereby enabling glove 11 to be readily fitted over the surgeon's hand.

The feel of improved surgical glove 11 upon the hand, thumb and fingers of the surgeon is substantially the same as the conventional latex rubber glove. By virtue of the nylon overlay material 31, the feel between the tip of thumb portion 15 and the tips of the finger portions 16-19 is different from that of latex rubber. Though somewhat subjective, this feel may be described as resembling, to some extent, the same feel as exists between the tips of the thumb and fingers of the surgeon when no glove is worn. The reason for this difference in feel is due to the differences between the surface texture of the high-density nylon overlay 31 and the surface texture of latex rubber.

By the simple expedient of reversing, i.e., turning surical glove 11 inside out, it is apparent that the feel can be readily changed. A right-hand glove becomes a left-hand glove with overlay material 31 now located within glove 11. The feel between the tip of the thumb and the tips of the fingers of reversed glove 11 becomes that of latex rubber, the material of which foundation glove 12 is composed.

Without reversing, the feel of improved glove 11 can be made substantially the same as that to which the surgeon is accustomed by applying a thin over-coat of latex rubber. This is accomplished by dipping glove 11, while still mounted upon form 10, into a bath of liquid latex, withdrawing glove 11 with its form 10 from the bath of liquid latex, drying the thin coat of liquid latex adhering to the outer surface of glove 11, and curing the attached thin coat of latex by heating. This thin over-coat of latex rubber, identified by the numeral 41 as the outside surface of glove 11 in Figs. 1-4, provides an hermetic seal over the entire outer surface of glove 11. Glove 11 now has an inside surface of latex rubber, i.e., foundation glove 12, and an outside surface of latex rubber, i.e., over-coat 41. Not only does latex over-coat 41 provide additional resistance to penetration, it also enhances the protection of both surgeon and patient from exposure to contamination.

In the event of a rupture or penetration of the hermetic seal provided by thin latex over-coat 41 covering front face 13 of glove 11, as might occur during the course of an operation, it has been observed that body liquids, such as blood, reaching overlay 31 will cause staining of overlay 31 which is visible through the semi-transparent, thin latex over-coat. This visual indication is of some importance to the operating surgeon and serves as a warning that may necessitate replacement of the glove whose hermetic seal has been compromised.

It will be appreciated that the high-density, tightly interwoven nylon cloth composed of a very large number of high-strength filaments above-identified is but one example of the type and nature of a class of polymer fibers or filaments suitable for overlay 31. An additional representative example is an interwoven material having a warp of 30 denier nylon yarn with 95.3 yarns per cm (242 yarns per inch) and a fill of 30 denier nylon yarn with 72.4 yarns per cm (184 yarns per inch). This woven material, available from Burlington Industries and identified as Style No. 55163, consists of twenty-six (26) filaments per yarn in both the warp and the fill. While somewhat thicker, heavier, and less pliable, this woven material has excellent resistance to penetration. It is apparent, of course, that an overlay consisting of two or more layers of high-density, tightly interwoven, high-strength filaments will further enhance resistance to penetration, although sacrificing tactile sensitivity and ease of finger manipulation.

The twenty and thirty denier yarns forming the nylon material of overlay 31 are composed of bundles of continuous filaments of high tensile strength. The cutting of overlay 31 in the shape of thumb 15, fingers 16-19, and palm portion 20 of glove 11 may expose the cut ends of the filaments in both the warp and the fill. To maintain the high tensile sterength inherent in each filament and to prevent sliding or slippage between adjacent filaments, the cut ends may be bonded one to the other by heat sealing. The preferred method by which heat sealing of the cut ends of the bundles of nylon filaments is accomplished is a process known as hot-knife sealing. This process achieves both cutting and sealing of the peripheral edges of overlay 31 by a simple procedure.

The improved surgical glove as above described provides an appreciable improvement in resistance to penetration by sharp-pointed instruments, possesses a touch and feel closely approaching that of conventional surgical gloves, affords increased protection to both physician and patient, and produces a visual indication in the event of rupture or penetration.

## Claims

1. A surgical glove comprising a foundation glove (12) composed of thin, resilient, elastic rubber having a front surface (13) and a rear surface (14), said foundation glove including stalls for the thumb (15), fingers (16-19) and palm (20) of the human hand and a cuff (21) for surrounding the wrist portion of the arm, said foundation glove providing a hermetic seal for covering the hand, and an overlay of thin pliable material (31) provided to a portion only of the foundation glove (12), characterised in that said overlay is a limp and non-elastic material composed of a very large number of tightly interwoven fibers of high strength, said overlay of thin, pliable and limp non-elastic material being shaped to conform to the front surface, but not to the rear surface, of the thumb, fingers and palm portions of said foundation glove, said shaped overlay of thin, pliable and limp material being attached or bonded to the front surface of the thumb, fingers and palm portions of said foundation glove, and having a resistance to penetration to sharp-pointed instruments at least three times greater than the resistance to penetration of the rubber forming the foundation glove (12).

2. A surgical glove as claimed in claim 1, characterised in that said overlay (31) is composed of a large number of tightly interwoven yarns of polymer material, said yarns of polymer material being comprised of bundles of high-strength polymer filaments.

3. A surgical glove as claimed in claim 2, characterised in that said overlay material of tightly interwoven yarns of polymer material has a warp of at least 79 yarns per cm (200 yarns per inch) and a fill of at least 70 yarns per cm (180 yarns per inch), the yarns in the warp and in the fill being composed of bundles of high-strength polymer filaments.

4. A surgical glove according to claim 2 or 3, wherein the polymer yarn is nylon yarn.

5. A surgical glove according to claim 2, 3 or 4, wherein the bundles comprise at least seven filaments per yarn.

6. A surgical glove as claimed in any preceding claim, wherein the overlay (31) material has a resistance to penetration of sharp, pointed instruments three to seven times greater than the resistance to penetration of the rubber material of the foundation glove (12).

7. A surgical glove as claimed in any preceding claim, characterised in that said overlay has a thickness of approximately 0.076 mm (0.003 in) and has a density of at least 157 yarns per cm² (400 yarns per in²).

8. A surgical glove as claimed in any preceding claim, wherein the overlay (31) is adhesively secured to the underglove (12).

9. A surgical glove as claimed in any preceding claim, characterised by a coat of thin, flexible, elastic and preferably semi-transparent material covering said shaped overlay of thin, pliable and limp material.

10. A surgical glove as claimed in claim 9, characterised in that the over-coat of thin, flexible and elastic material covers said foundation glove and its attached overlay, said over-coat forming an hermetic seal over the surface of and around the edges of said attached overlay.

## Patentansprüche

1. Operationshandschuh mit einem Unterlagenhandschuh (12), bestehend aus dünnem, federndem, elastischem Gummi mit einer vorderen Fläche (13) und einer hinteren Fläche (14), wobei der Unterlagenhandschuh Fingerlinge für den Daumen (15) und die Finger (16-19) sowie einen Handteller (20) einer menschlichen Hand und eine Manschette (21) zum Umschließen des Handgelenkabschnitts eines Armes umfaßt, und der Unterlagenhandschuh eine hermetische Abdichtung zur Abdeckung der Hand schafft, und einer für nur einen Teil des Unterlagenhandschuhs (12) vorgesehenen Auflage aus dünnem, geschmeidigem Material (31),
dadurch **gekennzeichnet**,
daß die Auflage ein biegsames und nichtelastisches Material ist, bestehend aus einer sehr großen Anzahl engverwobener Fasern hoher Festigkeit, wobei die Auflage des dünnen, geschmeidigen und biegsamen, nichtelastischen Materials mit der vorderen Fläche, jedoch nicht mit der hinteren Fläche des Daumenteils, der Fingerteile und des Handflächenteils des Unterlagenhandschuhs übereinstimmend geformt ist, die geformte Auflage des dünnen, geschmeidigen und biegsamen Materials an der vorderen Fläche des Daumenteils, der Fingerteile und des Handflächenteils des Unterlagenhandschuhs angebracht oder verbunden ist und einen mindestens dreimal so großen Widerstand gegen Eindringen eines scharfen, spitzen Instruments als der Widerstand gegen Eindringen des den Unterlagenhandschuh (12) bildenden Gummis hat.

2. Operationshandschuh nach Anspruch 1,
dadurch **gekennzeichnet**,
daß die Auflage (31) aus einer großen Anzahl engverwobener Garne eines Polymermaterials besteht, wobei die Garne des Polymermaterials aus Bündeln hochfester Polymerfäden bestehen.

3. Operationshandschuh nach Anspruch 2,
dadurch **gekennzeichnet**,
daß das Auflagematerial der engverwobenen Garne des Polymermaterials eine Kette von mindestens 79 Garnen pro cm (200 Garne pro Inch) und einen Schuß von mindestens 70 Garnen pro cm (180 Garne pro Inch) aufweisen und die Garne in der Kette und in dem Schuß aus Bündeln von hochfesten Polymerfäden bestehen.

4. Operationshandschuh nach Anspruch 2 oder 3,
dadurch **gekennzeichnet**,
daß das Polymergarn ein Nylongarn ist.

5. Operationshandschuh nach Anspruch 2, 3 oder 4, wobei die Bündel mindestens sieben Fäden pro Garn umfassen.

6. Operationshandschuh nach einem der vorhergehenden Ansprüche, wobei das Auflagematerial (31) einen Eindringwiderstand gegen scharfe, spitze Instrumente aufweist, der drei- bis siebenmal größer als der Eindringwiderstand des Gummimaterials des Unterlagenhandschuhs (12) ist.

7. Operationshandschuh nach einem der vorhergehenden Ansprüche,
dadurch **gekennzeichnet**,
daß die Auflage eine Dicke von ungefähr 0,076 mm (0,003 Inch) und eine Dichte von mindestens 157 Garnen pro cm² (400 Garne pro Inch²) aufweist.

8. Operationshandschuh nach einem der vorhergehenden Ansprüche, wobei die Auflage (31) adhäsiv an dem Unterlagenhandschuh (12) befestigt ist.

9. Operationshandschuh nach einem der vorhergehenden Ansprüche,
**gekennzeichnet** durch
eine Beschichtung aus einem dünnen, flexiblen, elastischen und vorzugsweise halbtransparenten Material, die die geformte Auflage aus dünnem, geschmeidigem und biegsamem Material bedeckt.

10. Operationshandschuh nach Anspruch 9,
dadurch **gekennzeichnet**,
daß die Überschicht aus dünnem, flexiblen und elastischen Material den Unterlagenhandschuh und seine angebrachte Auflage bedeckt, und daß die Überschicht eine hermetische Abdichtung der Oberfläche und rings um die Kanten der angebrachten Auflage bildet.

## Revendications

1. Gant chirurgical comprenant un gant de base (12) constitué d'une fine couche de caoutchouc élastique et résilient, définissant une surface externe (13) et une surface interne (14), ledit gant de base comprenant des logements pour le pouce (15), les doigts (16-19) et la paume (20) de la main, et une manchette (21) entourant la partie formant le poignet du bras, ledit gant de base formant une enveloppe étanche, hermétique recouvrant la main, une partie dudit gant de base étant associée à une couverture (31) à base d'une matière fine et pliable, caractérisé en ce que ladite couverture est à base d'une matière non élastique, sans consistance, composée d'un très grand nombre de fibres de haute résistance tissées entre elles de manière dense, ladite couverture constituée de ce matériau non élastique, fin, pliable et sans consistance, étant mise en forme pour recouvrir la surface externe, mais non la surface interne, des parties du gant de base recouvrant le pouce, les doigts et la paume, ladite couverture de matériau fin, pliable et sans consistance, étant fixée ou liée à la surface externe des parties du gant de base correspondant au pouce, aux doigts et à la paume, et ayant une résistance à la pénétration sous l'action d'instruments pointus au moins trois fois supérieure à la résistance à la pénétration du caoutchouc formant le gant de base (12).

2. Gant chirurgical selon la revendication 1, caractérisé en ce que ladite couverture (31) est constituée d'un très grand nombre de fils à base de polymère tissés entre eux de manière dense, lesdits fils étant eux-mêmes formés d'un faisceau de filaments en polymère à haute résistance.

3. Gant chirurgical selon la revendication 2, caractérisé en ce que la dite couverture constituée de fils à base de polymère tissés entre eux de manière dense, présente une densité en chaîne d'au moins 79 fils par centimètre (200 fils par pouce) et une densité en trame d'au inoins 70 fils par centimètre (180 fils par pouce), les fils de chaîne et les fils de trame étant constitués de filaments à base d'un faisceau de filaments en polymère à haute résistance.

4. Gant chirurgical selon l'une des revendications 2 ou 3, caractérisé en ce que le fil en polymère est un fil nylon.

5. Gant chirurgical selon l'une des revendications 2,3 ou 4, caractérisé en ce que chaque fil comporte au moins sept filaments.

6. Gant chirurgical selon l'une des revendications précédentes, caractérisé en ce que la couverture (31) présente une résistance à la pénétration d'instruments pointus trois à sept fois plus grande que la résistance à la pénétration du caoutchouc constituant le gant de base (12).

7. Gant chirurgical selon l'une des revendications précédentes, caractérisé en ce que la couverture a une épaisseur d'environ 0,076 mm (0,003 pouce) et a une densité d'au moins 157 fils/cm2 (400 fils par pouce carré).

8. Gant chirurgical selon l'une des revendications précédentes, caractérisé en ce que la couverture (31) est fixée par collage au gant de base (12).

9. Gant chirurgical selon l'une des revendications précédentes, caractérisé en ce que la couverture à base de matière fine, pliable et sans consistance, est recouverte d'une couche de matière élastique et de préférence semi-transparente.

10. Gant chirurgical selon la revendication 9, caractérisé en ce que la couche de matière fine, flexible et élastique, qui recouvre le gant de base et la couverture qui lui est associée, forme une couche hermétique étanche à la surface et autour des bords de ladite couverture.
